Europäisches Patentamt

European Patent Office

Office européen des brevets

(19)

(11) Publication number: **0 077 689**
A2

# (12) EUROPEAN PATENT APPLICATION

(21) Application number: **82305563.7**

(22) Date of filing: **19.10.82**

(51) Int. Cl.³: **C 12 N 15/00**
C 12 P 21/02, C 12 N 1/00
//C12R1/865

(30) Priority: **19.10.81 JP 167615/81**

(43) Date of publication of application:
**27.04.83 Bulletin 83/17**

(84) Designated Contracting States:
**AT BE CH DE FR GB IT LI LU NL SE**

(71) Applicant: **SUNTORY KABUSHIKI KAISHA**
**1-40, Dojimahama 2-chome**
**Kita-ku Osaka(JP)**

(72) Inventor: **Takano, Isamu**
**151-36, Sakurai Shimamoto-cho**
**Mishima-gun Osaka 618(JP)**

(72) Inventor: **Ono, Teiichi**
**2-2-812, Minase 2-chome Shimamoto-cho**
**Mishima-gun Osaka 618(JP)**

(72) Inventor: **Tanaka, Shoji**
**8-46, Minamikaneden-cho 1-chome**
**Suita Osaka 564(JP)**

(72) Inventor: **Oshima, Takehiro**
**3-4, Besshonakanomachi**
**Takatsuki Osaka 569(JP)**

(74) Representative: **Raynor, John et al,**
**W.H.Beck, Greener & Co 7 Stone Buildings Lincoln's Inn**
**London WC2A 3SZ(GB)**

(54) **Method of gene manipulation using an eukaryotic cell as the host.**

(57) A cell of an eukaryote such as a yeast is transformed with a gene in which the untranslatable 3' end portion of a structural gene is added downstream from an exogenous gene. The resulted transformant reveals far higher level of expression with the exogenous gene than the case of being not added with the 3' end portion.

EP 0 077 689 A2

./...

## FIG. I

```
              1    2    3    4    5    6    7    8    9    10
         Met  Tyr  Gly  Gly  Phe  Leu  Arg  Lys  Tyr  Pro  Lys  stop stop
Hind Ⅲ
←——F1——→ ←——— F2 ———→ ←—— F3 ———→ ←—— F4 ——→

AGCTT CATGT ATG GCGGT TTCCTT C CTAAGT ATC CGAA GT AATAG
   AGTACATAC CGCCA AAGGAA G CAT TCA TAG GCTT CATTAT C CTAG
   ←——————F8———————→ ←———————F7———————→ ←———————F6———————→ ←—F5——
                                                              BamHI
```

|       |                    |       |                    |
|-------|--------------------|-------|--------------------|
| F-1   | 5′ AGCTTCATGT      | F-5   | 5′ GATCCTATT 3′    |
| F-2   | ATGGCGGTTTCC       | F-6   | ACTTCGGATACT       |
| F-3   | TTCGTAAGTATC       | F-7   | TACCAAGGAAAC       |
| F-4   | CGAAGTAATAG        | F-8   | CGCCATACATGA       |

```
                         HindⅢ
            Pro  Glu │Ser  Phe  Met  Tyr
     ----- CCCGAA │AGC  TTC  ATG  T----
     ----- GGGCTT  TCG A│AG  TAC  A----
                        ↓
     ←————————————————→ ←————————————————→
          Trp1 gene          αNEgene
```

## Method of gene manipulation using an eukaryotic cell as the host

This invention relates to a method of gene manipulation using an eukaryotic cell as the host.

A large number of studies have so far been made on the gene manipulation technology using prokaryotic cells, typically Escherichia coli, as the hosts and it is reported that somatostatin, human growth hormone, insulin, interferons and so forth are being produced by the so-called recombinant DNA technique.

However, successful expression of exogenous genes in eukaryotic cells has been reported only for the rabbit $\beta$-globin etc. by SV40 but not for useful substances such as mentioned above.

The present inventors pursued researches on their judgement that, since the desired substances are primarily produced by eukaryotic cells, the use as host cells of eukaryotic cells having common gene structures and functions is more advantageous than the use of prokaryotic cells, typically Escherichia coli. They selected yeasts as the experimental models of eukaryotic cells on the grounds of easy conditions of cultivation, rapid cell division rate and easiness in handling. As a result of their intensive research works on the expression of exogenous genes in yeasts, they succeeded in said expression. This invention is based on this success.

In eukaryotic organisms, mRNAs generally carry polyadenylic acid called polyA added thereto. This polyA does not code for any amino acid but supposedly contributes to stabilization of mRNAs themselves and improvement in efficiency of translation into amino acids.

An eukaryotic cell DNA has a region which corresponds to the untranslatable 3' end portion of mRNA, and this region supposedly has some relation with the control of transcription from DNA to mRNA; for instance, it is suggested that the region has the function of addition of poly A.

The present inventors further pursued their researches on the region corresponding to such untranslatable 3' end portion and as a result they found that expression of an exogenous gene in an eukaryotic cell can be attained in good yield when the region corresponding to the untranslatable 3' end portion of a structural gene is added downstream from the exogenous gene. The present invention has been completed on the basis of this finding.

The object of the invention is to provide a new method for gene manipulation using an eukaryotic cell as the host.

Thus, the invention is directed to a method of gene manipulation using an eukaryotic cell as the host which comprises transforming an eukaryotic cell with a gene comprising the untranslatable 3' end portion of a structural gene as added down stream from the objective exogenous gene.

The promoter may be a gene derived from an eukaryotic cell. Thus, for example, when the host is a yeast, the TRP1, CYC-1, CYC-7, glyceraldehyde-3-phosphate dehydrogenase and other genes may be used.

The objective exogenous gene may be, for example, a chemically synthesized DNA or a cDNA obtainable from a mRNA by the use of reverse transcriptase.

For somatostatin, alpha-neoendorphin, calcitonin and insulin, for instance, the DNAs may be derived by chemical synthesis, whereas, for interferons, growth hormones, etc., the cDNAs may be derived by reverse transcription of mRNAs.

The addition of the region corresponding to the untranslatable 3' end portion of the structural gene to a site downstream from the exogenous gene can be performed by any known method.

Hereinafter, for brevity of description, the invention is described with regard to the exemplary case where the exogenous gene is the chemically synthesized alpha-neoendorphin (hereinafter abbreviated to α-N.E. ) gene.

α-N.E. is a new intracerebral hormone found in the swine hypothalamus in 1979 by Kangawa, Matsuo and others. Reportedly, it has the structure $H_2N$-Tyr-Gly-Gly-Phe-Leu-Arg-Lys-Tyr-Pro-Lys-COOH.

The present inventors synthesized the α-N.E. gene and on the other hand constructed a shuttle vector. They joined the α-N.E. gene with the shuttle vector. On that occasion, they added the region corresponding to the untranslatable
3' end portion lost in the course of the shuttle vector
construction downstream from the α-N.E. gene. At the same
time, a control was prepared without addition of such region.

A yeast (<u>Saccharomyces cerevisiae</u>) was transformed with
these α-N.E. gene-containing plasmid shuttle vectors and
the transformants were cultivated.  The α-N.E.-containing
hybrid protein thus produced in the culture broth was
harvested and decomposed with cyanogen bromide to give
α-N.E.  The yields were determined by radioimmunoassay and
it was revealed that the expression in the yeast carrying
the plasmid with the region corresponding to the untranslatable 3' end portion as added is about 10-fold as compared
with the yeast carrying the plasmid with no such region
added.

When <u>Escherichia coli</u>.K12 was transformed with the
above-mentioned plasmid, the expression was almost the same
irrespective of the addition or no addition of the region
corresponding to the untranslatable 3' end portion.

From the above results, it was established that the
addition of the region corresponding to the untranslatable
3' end portion of a structural gene downstream from the
objective exogenous gene is advantageous in the expression
of the exogenous gene, hence in the production of the desired
product.

The following example illustrates the invention in
more detail but is by no means limitative of the invention.

In the drawings, Fig. 1 shows an embodiment of the doublestranded polydeoxynucleotide containing the α-N.E. gene and to be used in the practice of the invention as well as oligodeoxyribonucleotides (F1 through F8) to be used in the synthesis of said embodiment. Fig. 2 shows the scheme for synthesizing completely protected bifunctional deoxyribonucleotides. Fig. 3 through Fig. 9 shows the scheme for constructing plasmids containing the α-N.E. gene.

Example

1. Chemical Synthesis of α-N.E. gene fragments

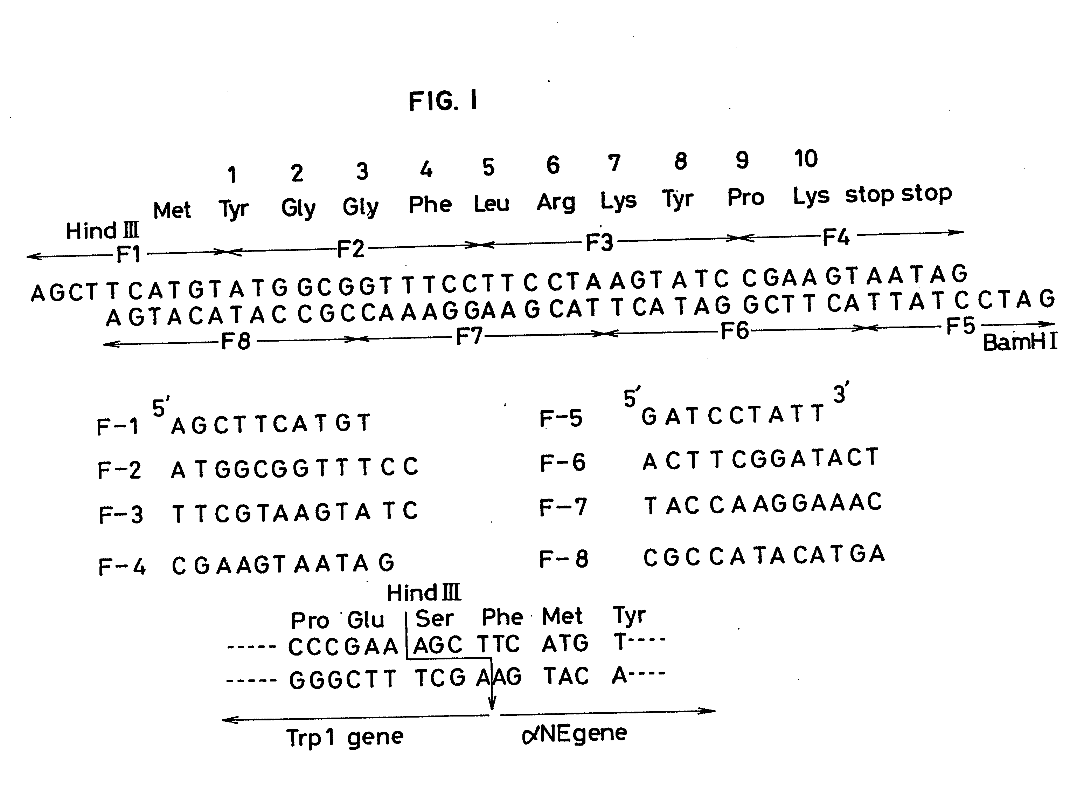

The methods and raw materials used for the chemical synthesis of eight oligodeoxyribonucleotides respectively named $F_1$ through $F_8$ as shown in Fig. 1 were in principle the same as those reported by Broka, Crea and others [Broka, C., et al. (1950), Nucl. Acids Res. 8, 5461 and Crea, R. et al. (1978), Proc. Natl. Acad. Sci. USA, 75, 5765] except for the modifications mentioned hereinbelow.

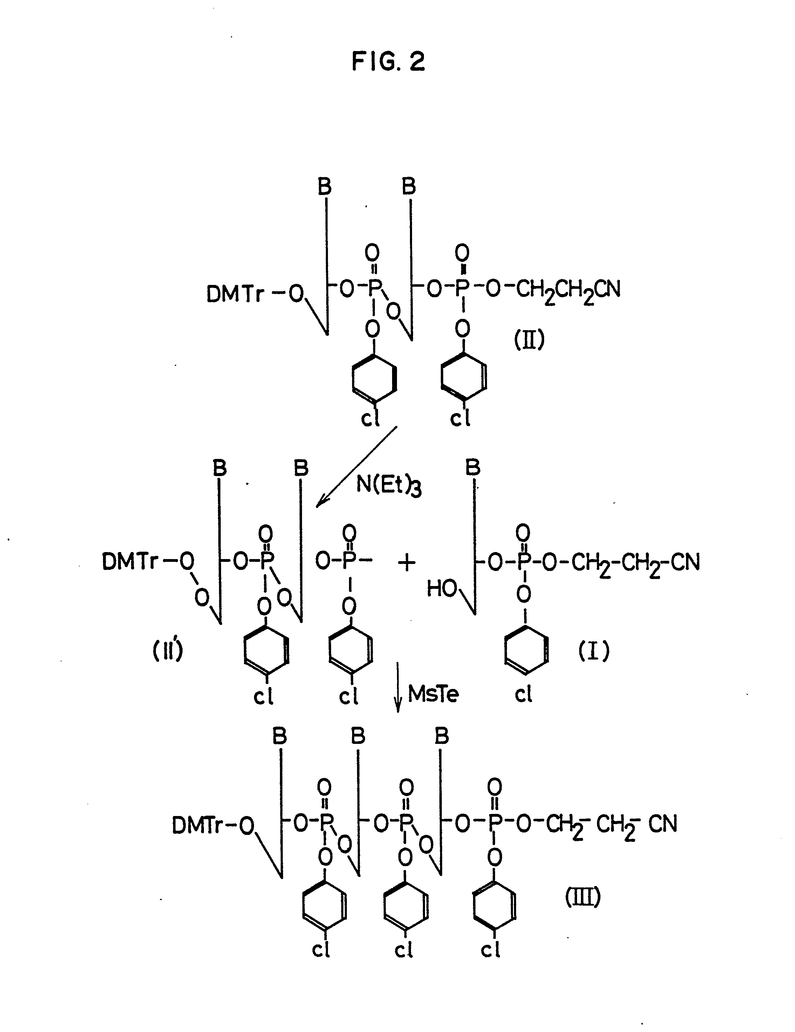

Completely protected bifunctional trimers (cf. Fig. 2) were synthesized on 3-5 millimole scales by the following modification. Thus, a completely protected bifunctional dimer II (one molar equivalent) was converted to the corresponding 3'-phosphoric acid diester component (II') with a pyridine-triethylamine-water (3:1:1 v/v) mixture, 5'-hydroxyl group component I (1.5 molar equivalents) was added thereto, and the condensation reaction was carried out with mesitylenesulfonyltetrazolide (MsTe, 2-3 molar equivalents) as the condensing agent. The product was isolated and purified by silica gel chromatography and $C_{18}$ reversed phase chromatography.

Starting with this group of compounds, eight oligodeoxyribonucleotides each having a specific base sequence, namely $F_1$ through $F_8$, were synthesized by the block condensation method.

Each of the eight fragments thus obtained was treated with concentrated $NH_4OH$ at 55°C for 10 hours and then with 80% acetic acid at room temperature for 10 minutes, whereby all the protective groups were eliminated. Purification of the end products was performed by high performance liquid chromatography (HPLC). The HPLC was conducted using an ALTEX model 110A liquid chromatograph. Each compound was isolated and purified with a PermaPhase AAX (du Pont) column by the linear gradient method using solvent A (0.005 M, $KH_2PO_4$, pH 4.0) and solvent B (0.05 M, $KH_2PO_4$- 1.0 M KCl, pH 4.0). Starting with solvent A, solvent B was added in an amount of 3% at minutely intervals for producing the gradient. Elution was carried out at 58°C at a flow rate of 2 ml per minute. Fractions covering each desired peak were collected, desalted by dialysis and lyophilized. Homogeneity of each of the eight completely deblocked oligodeoxyribonucleotides was confirmed by the fact that the above-mentioned two chromatographic procedures, namely ion exchange [PermaPhase AAX (du Pont)] column chromatography and reversed phase [μ-BondaPak $C_{18}$ (Waters)] column

chromatography each gave one single peak (cf. Table 1).

## Table 1

Retention time data for synthetic oligonucleotides

| | | Retention time (min.) | |
|---|---|:---:|:---:|
| Compound | Sequence | 1) | 2) |
| F1 | AGCTTCATGT | 7.7 | 11.4 |
| F2 | ATGGCGGTTTCC | 8.0 | 11.2 |
| F3 | TTCGTAAGTATC | 8.9 | 11.2 |
| F4 | CGAAGTAATAG | 8.6 | 11.1 |
| F5 | GATCCTATT | 6.8 | 11.4 |
| F6 | ACTTCGGATACT | 9.0 | 11.6 |
| F7 | TACGAAGGAAAC | 8.9 | 10.4 |
| F8 | CGCCATACATGA | 8.6 | 10.6 |

| | | |
|---|---|---|
| Packing | PermaPhase AAX | M BondaPak $C_{18}$ |
| Column | 0.21 x 50 cm | 0.4 x 25 cm |
| Solvent A | 0.005 M $KH_2PO_4$ (pH 4) | 0.01 M ethylenediamine-acetic acid (pH 7) |
| Solvent B | 0.05 M $KH_2PO_4$ (pH4)/ 1.0 M KCl | 0.01 M ethylenediamine-acetic acid (pH 7)/50% acetonitrile |
| Concentration gradient | Linear; concentration of B 0-99% over 33 minutes | Linear; concentration of B 0-50% over 20 minutes |
| Rate of flow | 2 ml/min. | 2 ml/min. |
| Temperature | 58°C | Room temperature |

The base sequence of each of the eight oligodeoxyribonucleotides was confirmed by labeling the 5' end with $[\gamma\text{-}^{32}P]$ATP using $T_4$ polynucleotide kinase followed by partial hydrolysis with nuclease and the two-dimensional mapping by electrophoresis at pH 3.5 using cellulose acetate and homochromatography using DEAE-cellulose [Bambara, J. E., et al. (1974), Nucl, Acids Res., 1, 331].

2.  Ligation of α-N.E. DNA fragments

After confirmation of the base sequences by the above procedure, the eight oligodeoxyribonucleotides were ligated using T4 DNA ligase by the following method.

Each of the fragments F2, F3, F4, F6, F7 and F8 was phosphorylated at the 5'-OH end with T4 polynucleotide kinase.

Thus, 200 µCi of $[\gamma\text{-}^{32}P]$ATP (7,400 Ci/mmole) was evaporated to dryness in an Eppendorff tube and reacted with 10 µg of a fragment in the presence of 10 units of T4 polynucleotide kinase in 60 µl of 50 mM Tris-HCl buffer (pH 8.0) plus 10 mM $MgCl_2$ plus 10 mM dithiothreitol (DTT) at 37°C for 20 minutes. Then, 10 nmoles of ATP and 10 units of T4 polynucleotide kinase were added to the above reaction mixture for phosphorylation of all the 5'-OH termini. The reaction was continued at 37°C for a further hour and then terminated by heating at 90°C for 5 minutes. The fragments F1 and F5 were not phosphorylated at the 5'-OH end so that two or more molecules thereof could not bind to the cohesive 5' end (HindIII and BamHI) sites from the objective gene
in the subsequent ligation reaction step. The unreacted
ATP was removed by placing 2.5 µg of each of the thus-obtained
phosphorylated fragments F2, F3, F4, F6, F7 and F8 and the
fragments F1 and F5 in a dialysis tube and performing dialysis
against one liter of water at 4°C overnight. Each dialysate
was adjusted to the final concentrations of 20 mM Tris-HCl
buffer (pH 7.6) and 10 mM $MgCl_2$, heated at 95°C for 2 minutes,
then allowed to stand at 80°C for 2 minutes, at 37°C for 10
minutes and at 25°C for 2 minutes, and submitted to the
following ligation reaction. The ligation reaction was
carried out at 11°C in the whole volume of 80 µl containing
20 mM Tris-HCl buffer (pH 7.6), 10 mM $MgCl_2$, 10 mM DTT and
0.4 mM ATP using 25 units of T4 DNA ligase. The size
marker used was the DNA fragment prepared by decomposing
φ×174 RF DNA with HinfI, dephosphorylating with bacterial
alkaline phosphatase and labeling with $[\gamma-^{32}P]$ATP and T4
polynucleotide kinase. Twenty-four hours after commencement
of the ligation reaction, the reaction mixture was heated at
65°C for 5 minutes to terminate the reaction. The reaction
mixture was electrophoresed on 15% polyacrylamide gel
containing 7 M urea with 90 mM Tris borate buffer (pH 8.4)
and 4 mM EDTA, followed by autoradiographic examination.
The desired α-N.E. DNA gene (45-mer nucleotide) was separated
from the unreacted fragments. The portion corresponding
to the 45-mer nucleotide was cut out from the gel, the lower end of the excised portion was placed in a dialysis tube, and electrophoresis was carried out in the above electrophoretic buffer diluted 10-fold. After the thus-effected elution of the DNA, the dialysis tube was dialyzed against water for desalting. The objective DNA was precipitated by addition of 3 volumes of ethanol. After separation by centrifugation, the DNA was dissolved in 20 µl of distilled water. The yield was 2.0 µg.

(3) Construction of the shuttle vector containing the Saccharomyces cerevisiae TRP1 gene

The replicon and selective marker in yeast (Saccharomyces cerevisiae) cells as used was the 3300 base pair DNA fragment obtained by HindIII digestion of pJDB219 [Beggs, J. D., Nature, 275 (1978), 104-108]. This fragment, which contained a portion of the yeast plasmid 2 µm DNA as well as the LEU2$^{+}$ gene, was made circular with T4 DNA ligase and used for transformation of the yeast. In the transformed yeast, this circular DNA was retained and caused expression of the LEU2$^{+}$ gene. Therefore, this 3300 base pair DNA fragment can be used as the replicon and selective marker (LEU2$^{+}$ gene) in yeast cells. This fragment was inserted into the Escherichia coli plasmid pBR322 at the HindIII cleavage site. This plasmid is a yeast-Escherichia coli shuttle vector for which the pBR322-derived replicon and the Ap$^{r}$ gene can be used as the selective markers in Escherichia coli and the 2 µm DNA replicon and the LEU2$^{+}$

gene can be used as the selective markers in the yeast. This plasmid was named pYE207.

The 850 base pair DNA fragment obtainable by EcoRI-BglII digestion of YRp7 contains the TRPI gene and the HindIII cleavage site found therein is suited for the insertion of the α-N.E. gene. Therefore, it is desirable that the plasmid has the HindIII cleavage site only on the TRP1 gene.

pYE 207 has two HindIII cleavage sites, one of which is to be removed on the occasion of inserting the 850 base pair DNA fragment containing the TRP1 gene of YRp7. Therefore, a plasmid, pYE227, was prepared in which the other HindIII cleavage site (nearer to the ampicillin resistance gene) was eliminated.

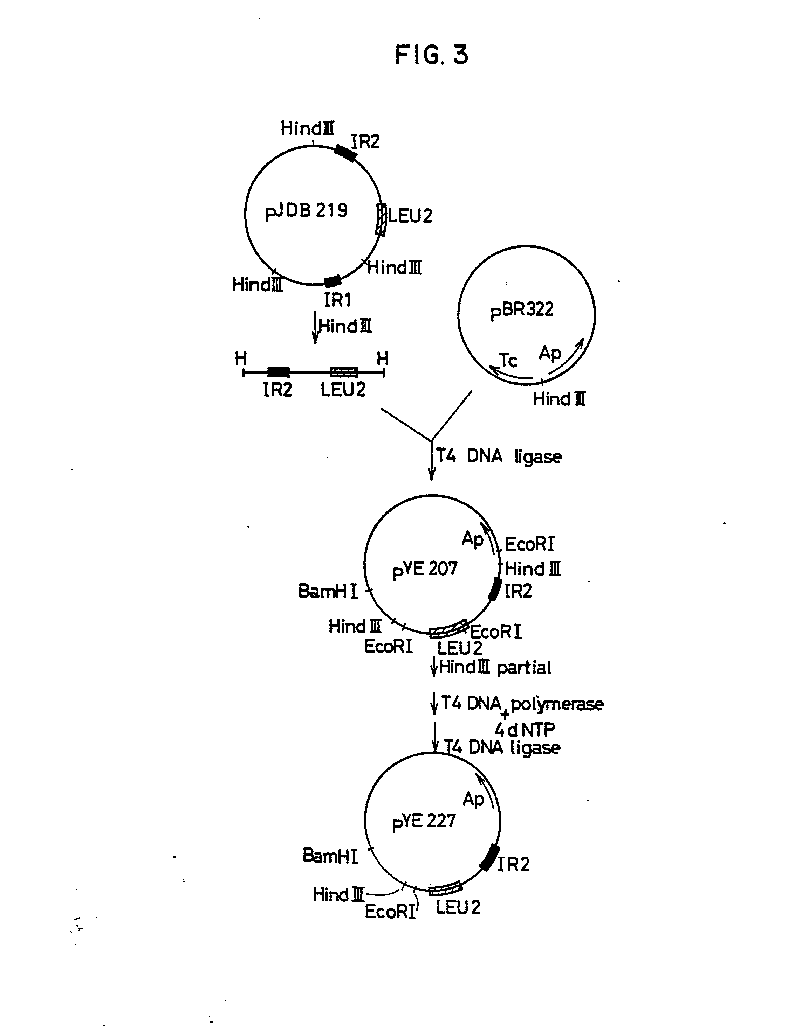

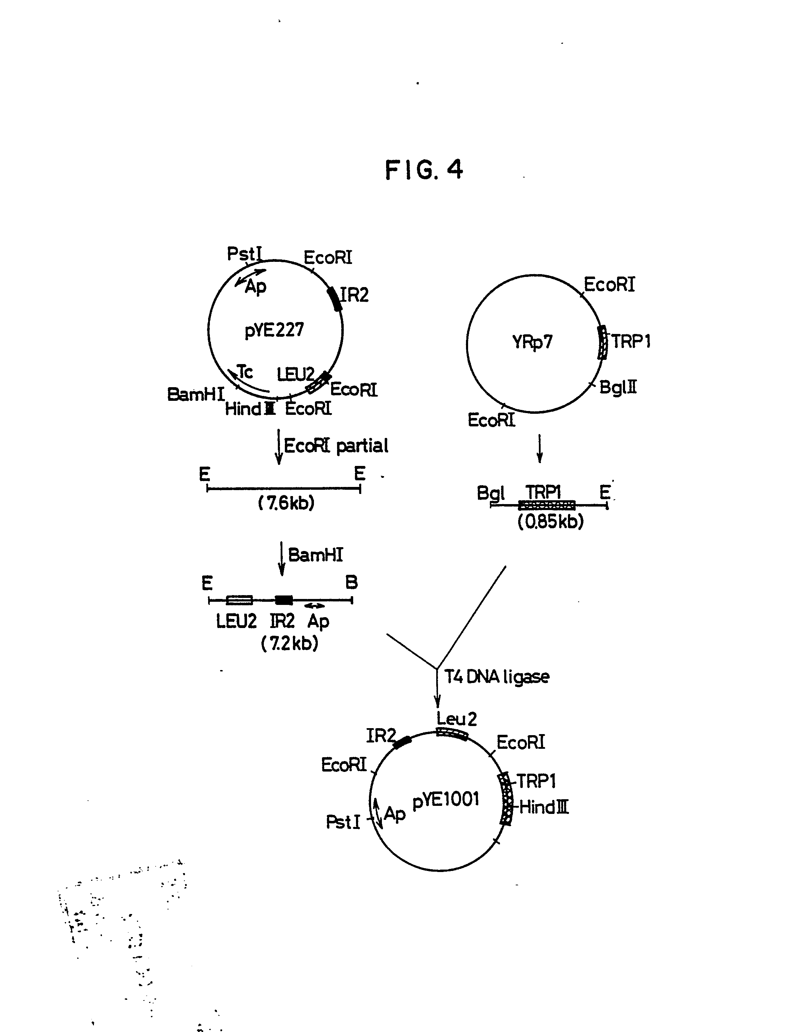

A plasmid, pYE1001, was prepared by inserting the 850 base pair DNA fragment obtained by EcoRI and BglII cleavage of YRp7 [Stinchcomb, D. T., et al., Proc. Natl. Acad. Sci. USA, 76, 1035-1039 (1979)] and containing the TRP1 gene between the EcoRI and BamHI cleavage sites of pYE227 shown in Fig. 3. This pYE1001 is a shuttle vector such as mentioned above and contains the yeast TRP1 gene. The following are the details (Fig. 3 and Fig. 4).

pJDB219 (5 µg) was completely digested by adding 10 units of HindIII thereto followed by warming at 37°C in TA buffer (33 mM Tris acetate, pH 7.9, 66 mM potassium acetate, 10 mM magnesium acetate, 0.05 mM dithiothreitol;

the one described by O'Farrell, P. H., et al. in MGG, 179, 421-435, 1980 minus bovine serum albumin) for an hour. The resulting fragments were separated by 0.8% agarose gel electrophoresis and stained with ethidium bromide. The gel portion corresponding to the 3,300 base pair fragment was cut out and the DNA fragment was eluted therefrom by electrophoresis. After extraction of the ethidium bromide from the eluate with phenol, the DNA was precipitated with 2 volumes of ethanol. Separately, 1 µg of pBR322 was completely digested with 2 units of HindIII by allowing the reaction to proceed at 37°C in TA buffer for an hour. Two volumes of ethanol was added to the digestion mixture for DNA precipitation. Both the precipitates were respectively dissoled in 10 µl of ligase buffer (20 mM Tris-HCl pH 7.5, 5 mM MgCl$_2$, 10 mM dithiothreitol, 1 mM ATP) and the solutions were combined. T4 DNA ligase (1 unit) was added and the reaction was allowed to proceed at 10°C for 18 hours. Two volumes of ethanol was added to the reaction mixture for DNA precipitation. The thus-obtained DNA was used as the donor plasmid for transformation of Escherichia coli. In this manner, a strain of Escherichia coli carrying pYE207 containing the above-mentioned 3,300 base pair DNA inserted in the HindIII site of pBR322 was obtained.

When a leuB mutant is used as the strain of Escherichia coli, the LEU2$^+$ gene of pYE207 is expressed in the Escherichia coli cell and the leucin dependency disappears. The transformant of Escherichia coli was cultivated and the cells were lysed with lysozyme and Triton X100. Cesium chloride (density 1.55 g/cm$^3$) and ethidium brimide (250 µg/ml) were added to the lysate solution obtained by centrifugation at 18,000 rpm for 30 minutes and the mixture was centrifuged at 45,000 rpm for 48 hours to give a plasmid DNA.

pYE207 (10 µg) was partially digested with 1 unit of HindIII by carrying out the reaction at 37°C in TA buffer for 15 minutes. The reaction was terminated by heating at 65°C for 10 minutes. Following separation by 0.8% agarose gel electrophoresis, a linear DNA corresponding to 7,600 base pairs was eluted from the gel and the DNA was recovered by precipitation with ethanol. The cohesive ends of this linear DNA as resulting from HindIII cleavage were rendered blunt by reacting with 0.33 mM each of dCTP, dATP, dTTP and dGTP in the presence of 0.1 unit of T4 DNA polymerase in T4 DNA polymerase buffer (67 mM Tris-HCl, pH 8.8, 6.7 mM MgCl$_2$, 10 mM mercaptoethanol, 16.6 mM ammonium sulfate, 167 µg/ml bovine serum albumin) at 37°C for 30 minutes. The reaction was stopped by heating at 65°C for 10 minutes. The DNA was recovered by ethanol precipitation and dissolved in 20 µl of T4 ligase buffer. Following addition of 2 units of T4 ligase, the blunt ends were joined together by reacting at 10°C for 18 hours.

There was obtained pYE227 with disappearance of the

<u>HindIII</u> cleavage site nearer to the Ap$^r$ gene of pYE207
(Fig. 3).

Using 2 units of EcoRI, 20 µg of pYE227 was partially
digested by carrying out the reaction in TA buffer at 37°C
for 15 minutes. This partial decomposition mixture was
separated by 0.8% agarose gel electrophoresis, and a
molecule (7,600 base pair linear DNA) resulting from
cleavage only at one site was recovered by the above-
mentioned method. 2 µg of this 7,600 base pair DNA was
completely digested with 15 units of <u>Bam</u>HI by carrying
out the reaction at 37°C in TA buffer for an hour. The
digestion mixture was separated by 0.8% agarose gel
electrophoresis, and a DNA corresponding to 7,200 base
pairs was recovered by the above-mentioned method.
Separately, 2 µg of YRp7 was treated with 3 units each
of <u>Bgl</u>II and <u>Eco</u>RI at 37°C in TA buffer to give a 850
base pair fragment, which was recovered by the above-
mentioned method. These two DNA fragments were dissolved
in 20 µl of T4 DNA ligase buffer, 1 unit of T4 DNA ligase
was added, and the reaction was allowed to proceed at
10°C for 18 hours. As in the above-mentioned case, a
shuttle vector, pYE1001, was obtained with the TRP1 gene
of YRp7 inserted in pYE227. The yeast was transformed
with this pYE1001. All the leucine-independent clones
showed tryptophan-independency and it was revealed that
the TRP1 gene was retained intact in the 850 base pair DNA fragment derived from YRp7.  pYE1001 was shown to be a
shuttle vector replicable also in <u>Escherichia coli</u> cells
(Fig. 4).

(4)   <u>Insertion of the chemically synthesized α-neoendorphin</u>
      <u>gene</u>

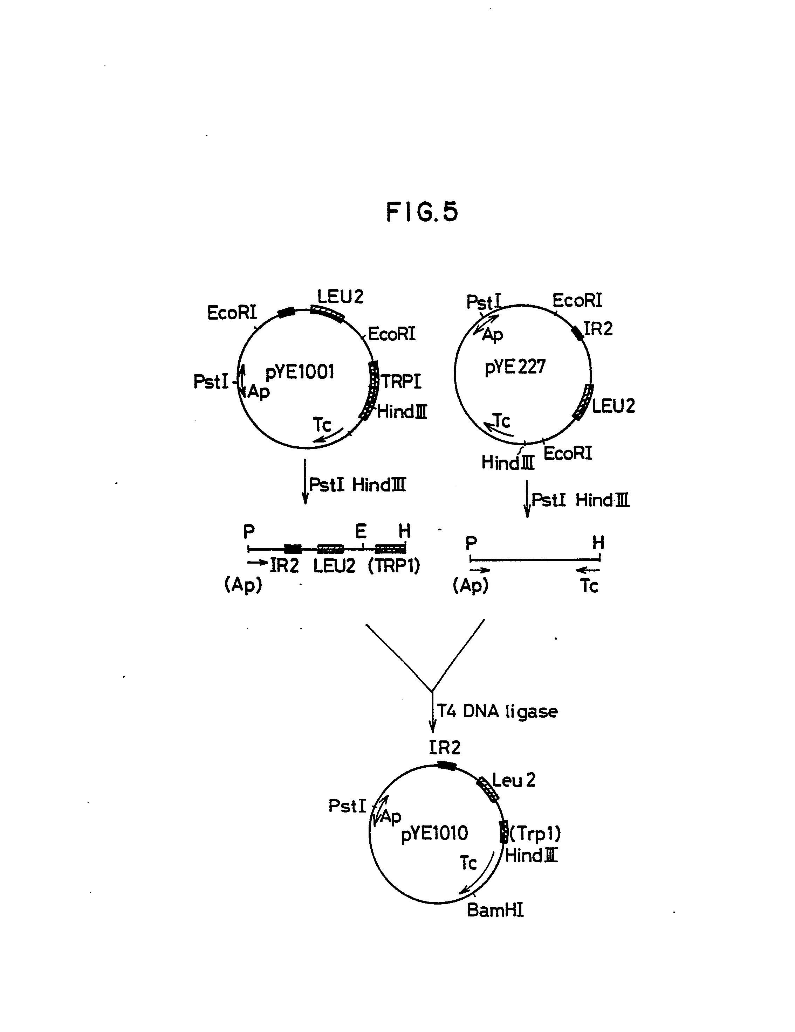

For facilitating the introduction of the above-mentioned
α-N.E. gene into the <u>HindIII</u> cleavage site on the yeast
TRP1 gene and for easy selection in <u>Escherichia coli</u>, there
was constructed a plasmid, pYE1010, containing the Tc$^r$ gene
of the plasmid pBR322 as introduced therein downstream
from the <u>HindIII</u> cleavage site on the TRP1 gene (Fig. 5).

Thus, 10 μg of pYE1001 was completely digested with
10 units each of <u>HindIII</u> and <u>SalI</u> by effecting the reaction
in TA buffer at 37°C for an hour.  A fragment corresponding
to 7,500 base pairs was isolated in the same manner as above
by electroeluting from agarose gel. Separately, 10 μg of
pBR322 was completely digested with 10 units each of
<u>HindIII</u> and <u>SalI</u> in TA buffer and a fragment corresponding
to 650 base pairs was obtained.  Both the fragments were
mixed and subjected to ligation in the same manner as above.
The thus-obtained pYE1010 showed tetracycline resistance
(5 μg/ml) in <u>Escherichia coli</u> and had one <u>HindIII</u> and one
<u>BamHI</u> cleavage site.  In the yeast, no TRP1 activity was
observed due to disappearance of the portion downstream
from the <u>HindIII</u> site on the TRP1 gene.

The 45-mer α-N.E. gene having the <u>HindIII</u> and <u>BamHI</u>

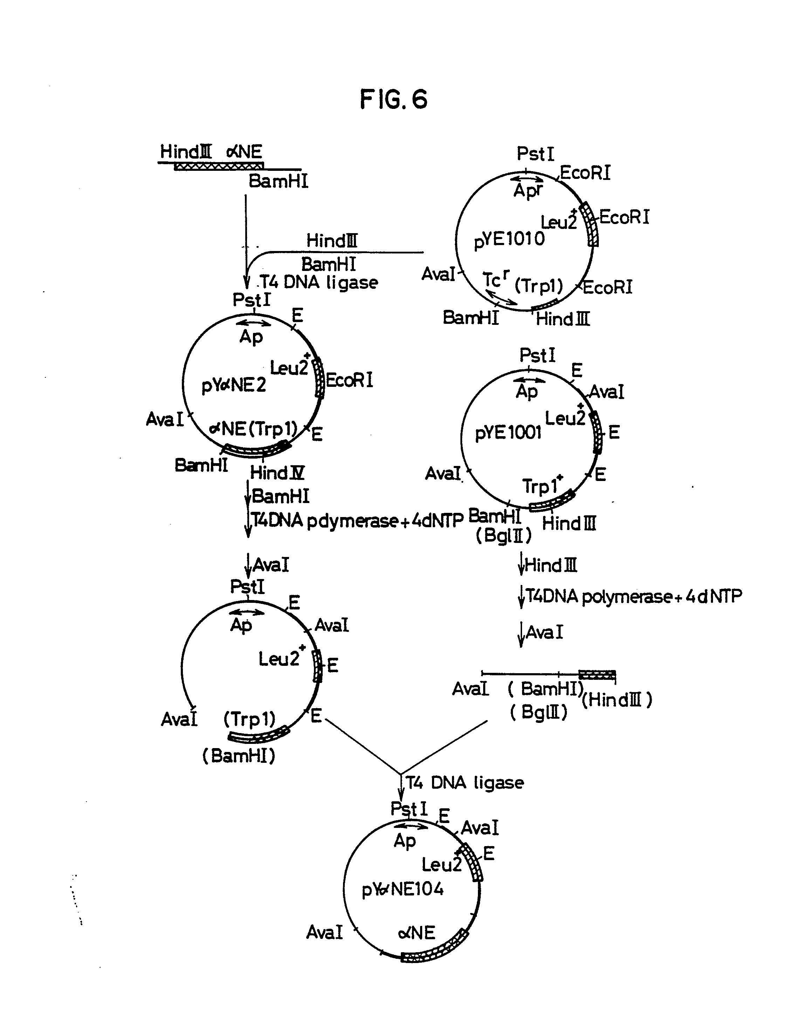

cohesive ends was introduced into this pYE1010 (Fig. 6).

Thus, 1.0 µg of the α-N.E. DNA prepared by the procedure mentioned above was heated at 95°C in 23 µl of a solution containing 20 mM Tris-HCl buffer, pH 7.6, and 10 mM MgCl$_2$ for 2 minutes and then cooled in an ice water bath, 1.2 nmoles of ATP, 6.5 units of T4 polynucleotide kinase and 10 mM DTT were added, and the reaction was conducted at 37°C for 20 minutes for phosphorylation of the 5'-end of the DNA. For phosphorylating all the DNA molecules at the 5' end, the reaction mixture was reheated at 95°C for 2 minutes and then cooled in ice water. Then, 100 mM DTT in a volume of one tenth of the liquid reaction mixture and 6.5 units of T4 polynucleotide kinase were added, and the reaction was further effected at 37°C for 20 minutes. The reaction mixture was then heated at 95°C for 2 minutes and cooled gradually to room temperature.

The plasmid pYE1010 DNA (5 µg) was completely digested with 5 units each of the restriction enzymes HindIII and BamHI, the resulting fragments were separated by 0.8% agarose gel electrophoresis, and the larger DNA fragment was recovered by electroelute. The fragment DNA (1 µg) thus obtained from pYE1010 by HindIII and BamH digestion and 0.1 µg of the α-N.E. gene DNA previously phosphorylated at the 5' end were dissolved in 30 µl of ligase buffer, and the reaction was allowed to proceed at 10°C for 18 hours using 1 unit of T4 DNA ligase. Two volumes of ethanol was added to the reaction mixture to give a DNA as a precipitate. This DNA precipitate was dissolved in 100 μl of a solution composed of 100 mM Tris-HCl buffer (pH 7.6), 6 mM $MgCl_2$ and 66 mM NaCl and the solution was used for transformation of E. coli JA 221. The transformant was cultured on a nutrient agar medium containing 40 μg/ml of ampicillin at 37°C overnight and the colonies obtained were replicated on a nutrient agar medium containing 5 μg/ml of tetracycline and tetracycline-sensitive colonies were selected. There were obtained six colonies as ampicillin-resistant, tetracycline-sensitive strains.

These independently isolated six strains were named JA221/pYαNE1, JA221/pYαNE2, JA221/pYαNE3, JA221/pYαNE4, JA221/pYαNE5 and JA221/pYαNE6, respectively. When the plasmid DNAs obtained from these strains were digested with the restriction enzymes HindIII and BamHI, these recombinant plasmids each gave two fragments. In all the six cases, the smaller fragment had the same size as the α-N.E. DNA prepared in vitro.

(5) Joining of the untranslatable 3' end portion of the yeast TRP1 gene

In eukaryotic cells, the untraslatable 3' end portion contains a region concerned with important functions such as addition of polyadenylic acid (PolyA). Since the yeast is also an eukaryotic cell, it is presumable that the untraslatable 3' end portion is of importance and that addition of a gene to this region possibly has an influence on the expression of the gene. Therefore, a plasmid comprising the 3' end portion DNA, which had been the latter half of TRP1 but removed in the step of constructing pYE1010, inserted immediately after the α-N.E. gene of pYαNE2 was constructed (Fig. 6).

Thus, 10 μg of pYαNE2 was completely digested with 20 units of BamHI in TA buffer at 37°C for an hour. The reaction was stopped by heating at 65°C for 5 minutes. Two volumes of ethanol was added for DNA precipitation.

The cohesive ends resulting from the BamHI digestion were filled by using DNA polymerase.

The BamHI DNA was dissolved in T4 DNA polymerase buffer, dCTP, dATP, dTTP and dGTP (each 0.33 mM) and 0.1 unit of T4 DNA polymerase were added, and the reaction was effected at 37°C for 30 minutes and then stopped by heating at 65°C for 10 minutes.

Separately, 10 μg of pYE1001 was completely digested by carrying out the reaction using 20 units of HindIII in TA buffer at 37°C for an hour and the resulting cohesive ends were filled by the use of DNA polymerase as in the above-mentioned case of pYαNE2. Using 10 units of AvaI, 25 μg of the linear pYαNE2 derived by digesting with BamHI and rendering the ends blunt was completely digested by carrying out the reaction in TA buffer for an hour, the products were separated by 0.8% agarose gel electrophoresis, and a 4,200 base pair fragment and a 2,400 base pair fragment were obtained in the same manner as mentioned above. Using 10 units of AvaI, 5 µg of the linear pYE1001 derived by digesting with HindIII and rendering the ends blunt was completely digested by conducting the reaction at 37°C for an hour, the products were separated by 0.8% agarose gel electrophoresis, and a 1,300 base pair fragment was obtained in the same manner as mentioned above. These three DNA fragments were mixed and, using 1 unit of T4 DNA ligase, the reaction was performed in the above-mentioned T4 DNA ligase buffer at 10°C for 18 hours. Two volumes of ethanol was added to the liquid reaction mixture for DNA precipitation. The thus-obtained DNA was used for transformation of E. coli by the above-mentioned method. From the thus-obtained ampicillin-resistant colonies, the plasmid DNA was separated and analyzed using restriction enzymes. In this manner, pYαNE101, pYαNE102, pYαNE103 and pYαNE104 each containing the TRP1 gene's latter half 3' end portion (250 base pairs) as inserted behind the BamHI cleavage site of pYαNE2.

(6)  Transformation of the yeast

Using the plasmid DNAs obtained by the above procedures, i.e. pYαNE2, pYαNE3, pYαNE101 and pYαNE104, as the transforming

DNAs, yeast transformation was carried out.

The transformants were selected as leucine-independent strains.

Thus, XS16-5C (a leu2 trpl his3) was shake-cultured in 200 ml of YPD medium containing 1% yeast extract, 2% polypeptone and 2% glucose at 30°C until the cell count of $2 \times 10^7$ cells/ml was attained. The cells were collected by centrifugation at 5,000 rpm for 5 minutes, washed once with 20 ml of 1.2 M sorbitol solution, and suspended in 20 ml of a solution composed of 10 mM EDTA, 25 mM DTT and 1.2 M sorbitol (pH 8.0). The suspension was warmed at 30°C for 10 minutes. Then, the cells were washed once with 20 ml of 1.2 M sorbitol and resuspended in 20 ml of a solution containing 0.1 mg/ml zymolyase 60K, 20 mM citrate buffer (pH 6.0) and 1.2 M sorbitol, and the reaction was coducted at 30°C for 30 minutes. The resulting protoplasts were washed with two 20-ml portions of 1.2 M sorbitol and then with one 20-ml portion of 1.2 M sorbitol containing 10 mM $CaCl_2$ and suspended in the same solution to a cell concentration of $10^9$ cells/ml. The transforming DNA (2 ug) was mixed with 0.2 ml of the protoplast suspension, and the mixture was allowed to stand at room temperature for 15 Minutes. Then, 2 ml of 30% polyethylene glycol 4000 containing 10 mM $CaCl_2$ and 10 mM Tris buffer, and the whole mixture was allowed to stand at room temperature for 20 minutes. The protoplasts were recovered by centrifugation at 3,000 rpm for 5 minutes and suspended in 2 ml of 1.2 M sorbitol solution containing 10 % polyethylene glycol 4,000. An adequate quantity (0.2-0.02 ml) of this suspension was placed on a plate medium containing 0.67% yeast nitrogen base (Difco), 2% glucose, 2% agar, 160 µg/ml tryptophan and 100 µg/ml histidine, and a regeneration medium (0.67% yeast nitrogen base, 2% glucose, 2% YPD and 3% agar) kept at 45°C was layered thereon. After cultivation at 30°C for 5 days, the resulting colonies were taken. In each case, the presence of the transforming plasmid DNA was confirmed in all the ten colonies taken. [Nucleic Acids Research, 4 1429 (1977)]

The yeast strain (XS16-5C) transformed with the plasmid pYαNE104 was named Saccharomyces cerevisiae. SBM304 and deposited in the Fermentation Research Institute, the Agency of Industrial Science and Technology (deposit number FERM BP-178).

(7)   Identification of α-N.E. by radioimmunoassay

The method of N. Minamino et al. [Biochem. Biophys. Res. Commun., in press] was used as the standard radio-immunoassay (RIA) method for α-N.E.

In yeast cells, α-N.E. is produced as a hybrid protein with the TRP1 gene product, and contains methionine at the N terminal. Decomposition of the hybrid protein with CNBr gives α-N.E. which is a decapeptide.

The yeast transformed with the pYαNE2, pYαNE3, pYαNE101

or pYαNE104 obtained in the above manner was cultivated in YPD for 18 hours, 1 ml of the culture broth ($5 \times 10^7$ cells/ml) was taken and centrifugated for cell recovery, and the cells were converted to protoplasts by effecting the reaction in 0.5 ml of 50 mM phosphate buffer containing 0.5 mg/ml zymolyase 60k and 10 mM β-mercaptoethanol at 30°C for 15 minutes.

The protoplasts were recovered by centrifugation and treated with 0.5 ml of 70% formic acid containing 5 mg/ml of cyanogen bromide for 24 hours. The reaction mixture was concentrated to dryness under reduced pressure, the residue was dissovled in 100 μl of 1 M acetic acid, and the solution was neutralized by adding 100 μl of 1.3 M Tris-HCl buffer, pH 8.4. To this solution was added 800 μl of RIS standard buffer (pH 0.4 phosphate buffer 50 mM, 0.25% bovine serum albumin, NaCl 80 mM, EDTA 25 mM) to make the total volume 1 ml. Then, 100 μl of the solution was taken and the activity was measured using the α-N.E. antibody essentially by the standard method of radioimmunoassay. The transformant Escherichia coli strain was cultivated in l-broth (0.5% yeast extract, 1% polypeptone, 0.5% NaCl, pH 7.5) for 18 hours, and the cells were recovered by centrifugation and directly treated with 70% formic acid containing 5 mg/ml of cyanogen bromide for decomposition of methionine. Thereafter, the activity was measured in the same manner as in the case of yeast.

The yields of α-N.E. in the yeast and the transformed Escherichia coli are shown in Table 2. While expression of the yeast TRP1 gene is attained also in Escherichia coli, the α-N.E. gene bonded to the TRP1 gene was also expressed in Escherichia coli cells. In the case of Escherichia coli, the α-N.E. gene expression was achieved in the transformants carrying the plasmids containing the latter half 3' end portion of the TRP1 gene as added (pYαNE101, pYαNE104) as well as in the transformants carrying the plasmids without no such portion added (pYαNE2, pYαNE3). On the other hand, in the yeast cells, the transformants carrying the plasmids with the latter half 3' end portion added showed about 10 times higher activity as compared with the case of no addition of such portion. The yield was 1 ng of α-N.E. per mg of total cell protein, this value corresponding to 2,500 molecules per yeast cell.

Table 2   Production of α-N.E. in yeast and in Escherichia coli

| Plasmid | αNE (pg)/protein (mg) | | Number of αNE molecules/cell | |
|---|---|---|---|---|
| | Yeast | E. coli | Yeast | E. coli |
| pYE1001 | 0 | 0 | 0 | 0 |
| pYαNE2 | 50 | 3510 | 130 | 280 |
| pYαNE3 | 55 | 6710 | 130 | 550 |
| pYαNE101 | 330 | 3300 | 305 | 270 |
| pYαNE104 | 1030 | 2780 | 2600 | 230 |

It was also noted that, in the transformed yeast carrying pYαNE101 or pYαNE104, the trpl mutation in the host was weakly reversed, whereas, in the case of pYαNE2 or pYαNE3, such fact was not observed.

This was investigated in terms of the growth rate in a tryptophan-deficient medium. The transformant yeast carrying pYαNE101 or pYαNE104 grew at a rate about one fifth as compared with that carrying pYE1001 (containing the whole TRP1 gene).

Since the TRP1-α-N.E. hybrid protein genes existing in these plasmids each contains two stop codons at the end of α-N.E. gene, the hybrid proteins produced can be regarded as one and the same. Therefore, it can be supposed that addition of the hind 3' end part of TRP1 leads either to efficient production of mRNA which is translated into the protein or to production of mRNA higher in stability. This tendency was observed also in another yeast strain (XP3-5A) other than XS16-5C. The stability of these plasmids in yeast cells was examined in terms of the frequence of appearance of leucine-dependent strains (presumably having lost the plasmids) after 10-generation culture in a unselective medium (80 µg/ml leucine-containing minimum medium or YPD). In the pYαNE104 transformant, the stability was not less than 95%. A similar result was obtained with pYαNE2.

(8) <u>Confirmation of high expression by addition of the un-</u>
<u>translatable 3' end portion</u>

As shown in Table 2, remarkable increase in the yield of *α*-N.E. was observed by adding the untranslatable 3' end portion of TRP1 structural gene to the down stream from *α* -N.E. gene. In order to confirm the effect, it was tried to join the untranslatable 3' end portion of a gene derived from a yeast, besides TRP1 gene, with the down stream from TRP1-*α*NE gene. As a gene to provide the untranslatable 3' end portion, the untranslatble 3' end portion of a structural gene coding the glyceroaldehyde-3-phosphate dehydrogenase (hereafter abbreviated as GAP-DH) of a yeast (<u>Saccharomyces</u> <u>cerevisiae</u>) was employed.

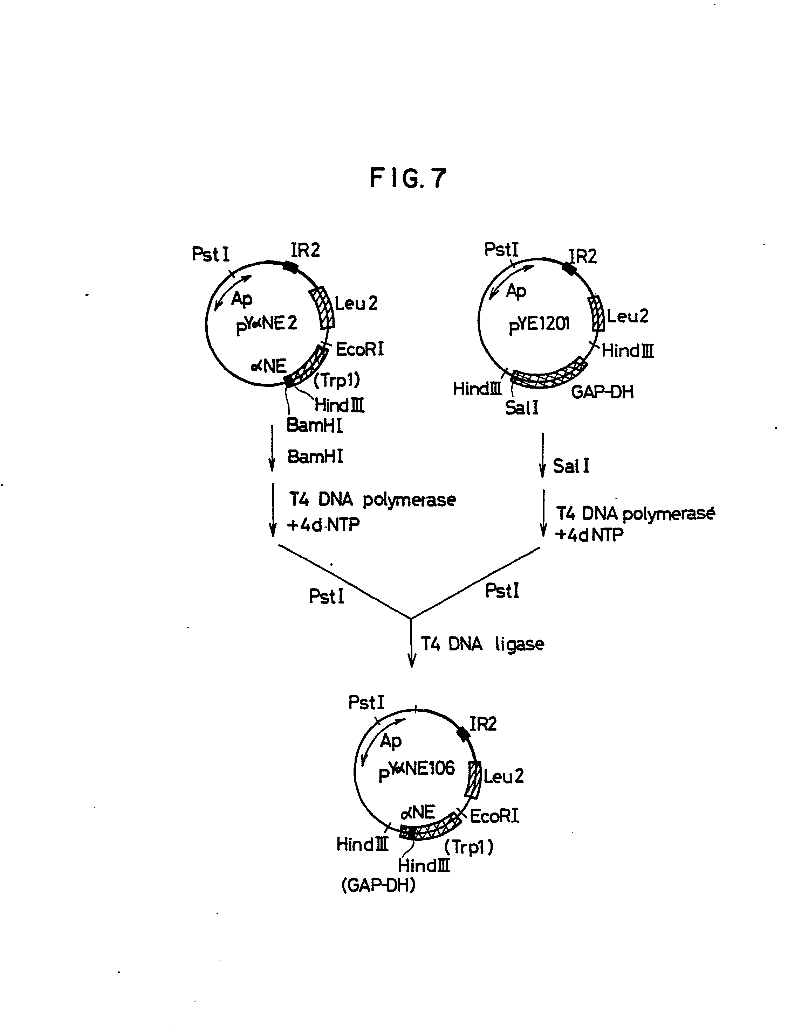

As detailed below, increased amount in the production of *α*-N.E. was observed to reach 10 times by addition of the untranslatable 3' end portion as compared with the case without the addtion. In the following, a method for prepar- ing a plasmid (pY*α*NE106) wherein the untranslatable 3' end portion of GAP-DH gene is added from the down stream of TRP1-*α*NE gene of pY*α*NE2 (Fig. 7) and an experiment of *α*-N.E. production in the transformed yeast are described. The yeast strain (XS16-5C) trnsformed with pY*α*NE106 was named <u>Saccharomyces</u> <u>cerevisiae</u> SBM 306 and deposited in the Fermentation Research Institute, the Agency of Industrial Science and Technology (deposit number FERM BP 176).

After 10 μg of pYαNE2 was cleaved with 30 units of BamHI, the cohesive ends resulted from the BamHI cleavage were filled in by a reaction using T4 DNA polymerase in T4 DNA polymerase buffer at 37° C for 30 minutes, while the 3' end portion of GAP-DH gene was obtained from pYE1201. After 10 μg of pYE1201 was cleaved with 30 units of SalI, the cohesive ends resulted from the SalI cleavage were filled in by a reaction using 1 unit of T4 DNA polymerase in T4 DNA polymerase buffer at 37° C for 30  minutes. And then, the respective DNA thus ontained was cleaved with 30 units of PstI and electroeluted from agarose gel to separate and purify a fragment containing α-N.E. gene from pYαNE2 and a fragment containing the 3' end portion of GAP-DH gene. The both fragments were joined together by a ligation reaction using 5 units of T4 DNA ligase in 20 μl of T4 DNA ligase buffer at 15° C for 17 hours.

Using 10 μl of the reaction mixture, E. coli was transformed, and, from ampicillin resistant transformants thus obtained, a plasmid was separated and analyzed to obtain pYαNE106. And then pYαNE106 was transformed into Saccharomyces cerevisiase XS16-5C by the method described in item (6). The obtained transformants were shake-cultured in  YPD medium (1 % yeast extract, 2 % polypeptone and 2 % glucose) at 30° C for 24 hours and the cells were harvested by centrifugation. To the cell pellet obtained from 1 ml of culture  medium, 0.5 ml of cooled acetone was added, allowed to stand at -20° C for 1 to 24 hours and freeze-dried. The dried cells were suspended in 70% formic acid solution containing 5mg/ml of cyanogen bromide and reacted in a dark place for 24 hours. After the reaction mixture was freezedried, $\alpha$-N.E. was eluted with 0.1 ml of 0.1N acetic acid and the eluate was neutralized with 0.1 ml of 1.3 M Tris-HCl to provide a sample for RIA. RIA was conducted according to the method of N. Minamino et al. The result of RIA is shown in Table 3. It was revealed that $\alpha$-N.E. gene expression in pY$\alpha$NE106 in which the 3' end portion of GAP-DH gene was added is almost the same extent as in pY$\alpha$NE104 in which the 3' end portion of TRP1 gene was added and almost ten times as much as in pY$\alpha$NE2 in which the 3' end portion was not added. In the untranslatable 3' end portion of GAP-DH gene, there is a base sequence of AATAAA which is considered to have some relation to the addition of poly A (polyadenylic acid) to mRNA in eukaryote. And it is considered that the addition of poly A to mRNA in eukaryote has a relation to the stability of mRNA and the efficency of translation to protein.

Therefore, it was expected to express more highly $\alpha$-N.E. gene by the addition of the 3' end portion (having a base sequence of AATAAA) of GAP-DH gene than that of the 3' end portion (not having a base sequnce of AATAAA) of TRP1 gene (pY$\alpha$NE104).

But the effect of the both additions were about 10- fold increase in the expression, samely as in the addition of the 3' end portion of TRP1 gene, as compared with the case having no addition.

Table 3

The effect on $\alpha$-N.E. yield by addition of the 3' end portion of GAP-DH

| Plasmid | Number of | $\alpha$-N.E. yield | |
|---|---|---|---|
| | cells/ml | pg/ml | molecules/cell |
| pY$\alpha$NE2 | $7.0 \times 10^7$ | 110 | $0.78 \times 10^3$ |
| pY$\alpha$NE104 | $6.8 \times 10^7$ | 1020 | $7.4 \times 10^3$ |
| pY$\alpha$NE106 | $9.0 \times 10^7$ | 1230 | $6.8 \times 10^3$ |
| | $8.5 \times 10^7$ | 1220 | $7.1 \times 10^3$ |

(9) Production of $\alpha$-N.E. by GAP-DH gene promoter

GAP-DH gene is a gene coding glyceroaldehyde-3-phosphate dehydrogenase (GAP-DH), a glycolysis enzyme.

It is considered that a strong promoter exists in GAP-DH gene because GAP-DH is produced in yeast in a great amount. Therefore, utilizing the promoter of GAP-DH gene, the production of $\alpha$-N.E. was tried. Furthermore, by the addition of the untranslatable 3' end portion of GAP-DH structural gene to the down stream from a heterogenous gene ($\alpha$-N.E. gene in this invention), an increase in $\alpha$-N.E.

**0077689**

yield was observed amounting nine times as much as the case without the addition.

Details of the experiments are described in the following.

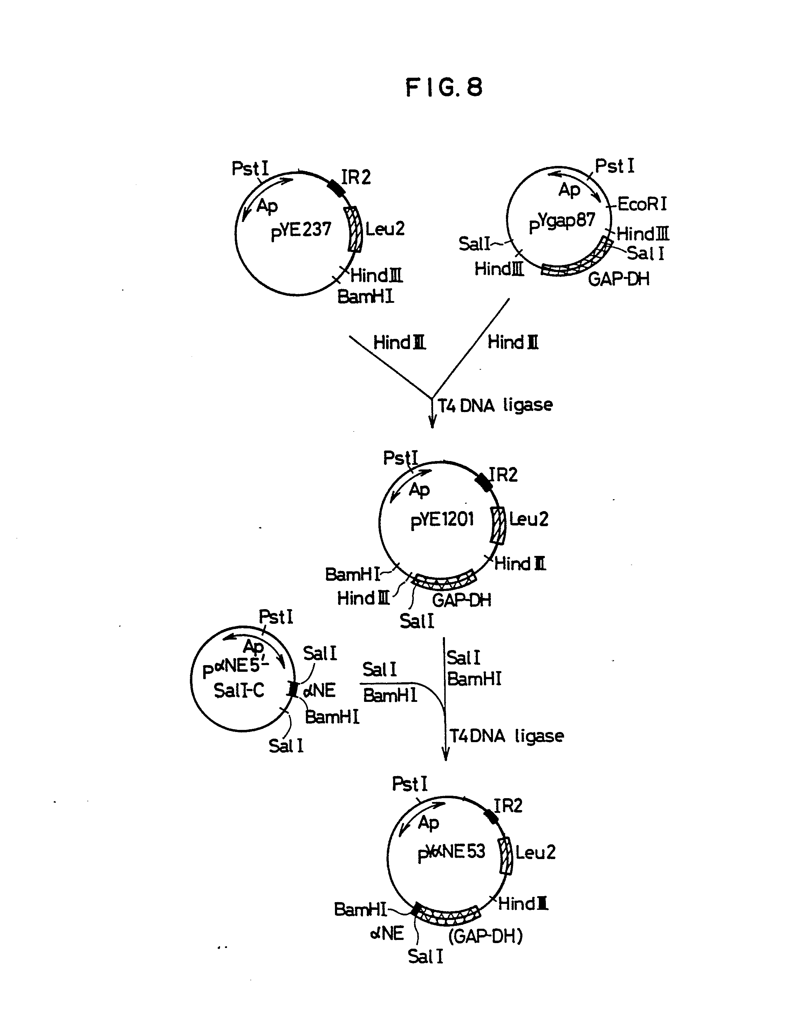

GAP-DH gene was already cloned by Holland et al. (J. P. Holland and M.J. Holland, J. Biol. Chem. 254: 9839 (1979)). These inventors obtained a plasmid pYgap87 carrying the GAP-DH gene cloned to pBR322 from a gene bank prepared by digesting (chromosomal genes of) Saccharomyces yeast XS16-5C with restriction enzyme HindIII. The cloning of GAP-DH gene was confimed in comparison with the data of Holland et al. A SalI fragment of plasmid p NE5' having $\alpha$ -N.E. gene and being already obtained was joined to SalI cleavage site existing near the C-terminus end of GAP-DH structural gene so as to match in  reading frame and the joined plasmid DNA was added by a part of pBR-322, yeast LEU2 gene and a part of 2 $\mu$m DNA to obtain E. coli - Yeast shuttle vector plasmid pY NE53 (Fig. 8).

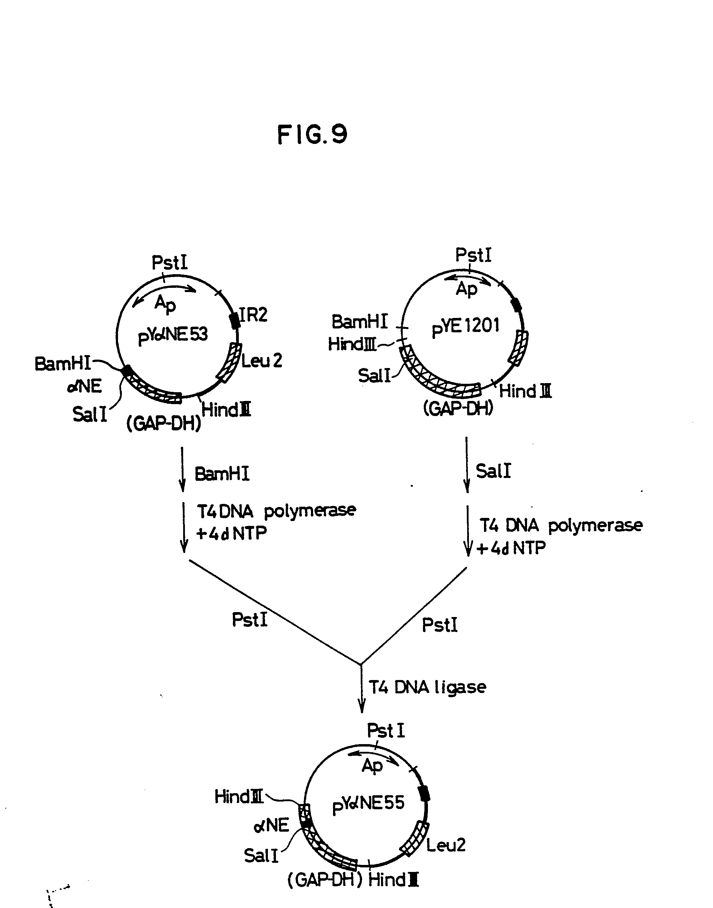

To the down stream from $\alpha$-N.E. gene existing in the pY$\alpha$NE53, the untranslatable 3' end portion of GAP-DH gene was added to prepare plasmid pY$\alpha$NE155 (Fig. 9). The both plasmids thus obtained were transformed into Saccharomyces yeast XS16-5C and then the productivity of $\alpha$-N.E. was invenstigated (Table 4).

As the result, a very high production of $\alpha$-N.E. (mean about 2 x $10^6$ molecules per cell) was shown with plasmid

pYαNE53 being not added by the untranslatable 3' end portion, however, mean about nine times as much as the above production was shown with the plasmid added by the untranslatable 3' end portion, therefore, the effect of adding the untranslarable 3' end portion of the structural gene is clearly observed.

The details of preparation methods for plasmid pYEα 1201 (a plasmid having GAP-DH gene and not having α-N.E. gene), pYαNE53 and pYαNE155, and production experiment for α-N.E. with Saccharomyces yeast transformed with each of the above-mentioned plasmids are described in the following.

The yeast strain (XS16-5C) transformed with pYαNE155 was named <u>Saccharomyces cerevisiae</u> SBM 355 and depositied in the Fermentation Research Institute, the Agency of Industrial Science and Technology (deposit number FERM BP 177).

Five $\mu$g of pYE237 (a shuttle vector which contains LEU2$^{+}$ gene as the selective marker and a portion of 2 $\mu$m DNA as a replicon in yeast, and, as the selective marker (Ap gene) and replicon in <u>E. coli</u>, contains a portion of pBR322 having one HindIII cleavage site and no SalI cleavage site) was reacted in TA buffer (33 mM Tris-HAc, pH 7.9, 66 mM potassium acetate, 10 mM magnesium acetate, 0.5. mM DTT) at 37° C for 1 hour employing 20 units of HindIII. Hereinafter, the reaction of a restriction enzyme was carried out in TA buffer at 37° C for 1 hour. Meanwhile, 5

$\mu$g of the plasmid pYgap87 already obtained was cleaved with 20 units of HindIII. From the resultant was separated 2.1 Kb DNA fragment (GAP-DH gene) by agarose electorphoresis and the DNA was eluted from a cut of agarose to be purified. This 2.1 Kb DNA fragment and the HindIII cleavage fragment of pYE237 were dissolved in 20 $\mu$l of DNA ligase buffer (20 mM Tris-HCl, pH 7.5, 10 mM $MgCl_2$, 10 mM DTT, 0.5 mM ATP), added with 1 units of T4 DNA ligase and reacted at $15^{\circ}$ C for 16 hours. Ten $\mu$l of the reaction mixture was added to 0.3 ml of E. coli JA221 (suspension) pretreated with $CaCl_2$ to proceed tramsformation.

From ampicillin-resistant transformants , a plasmid DNA was separated and analyzed by usual manner to obtain pYE1201 (Fig. 8)

Five $\mu$g of pYE1201 was cleaved to two fragments with 20 units of BamHI and 20 units of SalI, and after separating the fragments by agarose electrophoresis, the larger fragment was eluted from the agar and purified. While, the fragment containing $d$-N.E. gene was cleaved with 100 units of BamHI and 100 units of SalI, the resulted mixture was sepatated by an electorhoresis with 5 % polyacrylamide gel, and the DNA fragment corresponding to 50 base pairs was eluted and purified. The both fragments mentioned above were dissolved in 20 $\mu$l of DNA ligase buffer and reacted with 2 units of T4 DNA ligase at $15^{\circ}$ C for 16 hours.

10 $\mu$l of the reaction mixture was added to 0.3 ml of E. coli JA221 (suspension) pretreated with CaCl₂ to proceed transformation. From ampicillin-resistant transformants, a   plasmid DNA was separated and analyzed by usual manner to obtain pYαNE53.

To the pYαNE53 the 3' end portion of GAP-DH gene was added as follows (Fig. 9).

Ten μg of pYαNE53 was cleaved with 30 units of BamHI, reacted in a buffer (T4 DNA polymerase buffer) containing 67 mM Tris-HCl (pH 8.8), 6.7 mM MgCl₂, 10 mM 2-mercaptoethanol, 16.6 mM ammonium sulfate, 6.7 μM EDTA and four kinds of  dNTP at 37° C for 30 minutes employing 1 unit of T4 DNA polymerase to fill in the cohesive end formed by BamHI cleavage.

Meanwhile, the 3' end portion of GAP-DH gene was obtained from pYE1201.

Ten μg of pYE1201 was cleaved with 30 units of SalI and reacted in T4 polymerase buffer at 37° C for 30 minutes employing 1 unit of T4 DNA polymerase to fill in the cohesive end formed by SalI cleavage.

The resulted each plasmid DNA was cleaved with 30 units of PstI and a fragment containing α-N.E. gene separated from pYαNE53 DNA and a fragment containing the 3' end portion of GAP-DH gene from pYE1201 DNA were purified by electroeluting from agarose gel.

The both fragments were joined by reacting in 20 μl of T4 DNA ligase buffer at 15° C for 17 hours employing 5

units of T4 DNA ligase. A transformant was obtained using 10 $\mu$l of the reaction mixture according to the method mentioned above and, from the transformant, a plasmid DNA was separated and analyzed to obtain pY$\alpha$NE155.

Thus obtained plasmids, pYE1201, pY$\alpha$NE53 and pY$\alpha$NE155 were transformed into a yeast (Saccharomyces cerevisiae XS16-5C) according to the method described in item (6). The resulted reansformants were cultured in YPD medium (1 % yeast extract, 2 % polypeptone and 2 % glucose) at 30° for 24 hours and the cells were collected by centrifuging.

To the cell pellet obtained from 1 ml of the culture, 0.5 ml of cooled acetone was added, allowed to stand at -20° C for 1 to 24 hours and then freeze-dried. The dried cells were suspended in 70 % formic acid solution containing 5 mg/ml of cyanogen bromide and reacted in dark place for 24 hours. After the reaction mixture was freeze-dried, $\alpha$-N.E. was eluted with 0.1 ml of 0.1N acetic acid and the eluate was neutralized with 0.1 ml of 1.3 M Tris-HCl (pH8.0) to provide a sample for RIA. RIA was conducted according to the method described by N. Minamino et al. (Biochem. Biophys. Res. Commun. in press). The results are shown in Table 4.

As a hybrid protein in which $\alpha$-N.E. is joined at the N-terminus of the 323 amino acid residues, 2,000,000 molecules of $\alpha$-N.E. per cell were revealed in the case of pY$\alpha$NE53 (not having the 3' end protion of GAP-DH gene), and in the case of pYαNE155 (being added with the 3' end portion of GAP-DH gene), 20,000,000 molecules of α-N.E. per cells which corresponds to ten times of that of the above pYαNE 53. The production of α-N.E. in the culture medium was found to reach 2 μg/ml.

According to the stability investigation of the plasmids, after 10-generation culture in non-selective condition (cultivation in YPD medium) the stability was 10 to 20 %, being considerably unstable, in the case of pYE1201 (a plasmid having complete GAP-DH gene), however, in the cases of pYαNE53 and pYαNE155 (plasmids being joined with α-N.E. gene to the vicinity of the C-end of the GAP-DH gene), the stability was improved to some extent (Ref. Table 4).

Table 4


Productivity of $\alpha$-N.E. by GAP-DH promoter


| Plasmid | Number of cells/ml | $\alpha$-N.E. yield | | Stability of plasmid (%) |
|---|---|---|---|---|
| | | ng/ml | molecules /cell | |
| pYE1201 | $1.83 \times 10^8$ | $< 0.01$ | — | 15.1 |
| pY$\alpha$NE53 | $1.28 \times 10^8$ | 445 | $1.73 \times 10^6$ | 42.0 |
| " | $0.6 \times 10^8$ | 337 | $2.8 \times 10^6$ | 78.9 |
| " | $1.12 \times 10^8$ | 368 | $1.6 \times 10^6$ | N.D.* |
| pY$\alpha$NE155 | $0.99 \times 10^8$ | 2049 | $1.0 \times 10^7$ | 43.2 |
| " | $0.20 \times 10^8$ | 702 | $1.7 \times 10^7$ | N.D.* |
| " | $0.25 \times 10^8$ | 1260 | $2.5 \times 10^7$ | 25.5 |
| " | $0.42 \times 10^8$ | 1706 | $2.0 \times 10^7$ | N.D.* |


\* not determined.


The findings described in the examples mentioned above (ref. Tables 2, 3 and 4) strongly imply that the untranslatable 3' end portion of a structural gene, which seems to include base sequence coding the termination of transcription and the addition of poly A, performs an important role in the effective expression of a gene, for example, $\alpha$-N.E. gene in the above examples, in an eukaryotic cell.

-37-

Claims:

1. A method of gene manipulation using an eukaryotic cell as the host which comprises transforming an eukaryotic cell with a gene comprising the untranslatable 3' end portion of a structural gene as added downstream from an exogenous gene desired to be expressed.

2. A method according to claim 1 wherein the eukaryotic cell is the cell of a yeast.

3. A method according to claim 1 wherein the eukaryotic cell is the cell of a microorganism belonging to Sacchromyces.

4. A method according to claim 1 wherein the exogenous gene is a chemically synthesized DNA or a cDNA obtained from a mRNA by the use of reverse transcriptase.

5. A method according to claim 1 wherein the exogenous gene is somatostatin, alpha-neoendorphin, calcitonin, insulin, an interferon or a growth hormone.

6. A method according to claim 1 wherein a yeast is transformed with a gene prepared by joining alpha-neoendorphin with a shuttle vector and adding the region corresponding to the untranslatable 3' end portion lost in the course of construction of the shuttle vector downstream from the alpha-neoendorphin gene.

7. A cell derived by the transformation of a eukaryotic cell with a gene comprising the untranslatable 3' end portion of a structural gene as added downstream of an exogenous gene desired to be expressed.

8. A product derived from a cell as claimed in Claim 7, or a method as claimed in any one of Claims 1 to 6.

# FIG. I

|   | 1 | 2 | 3 | 4 | 5 | 6 | 7 | 8 | 9 | 10 | | |
|---|---|---|---|---|---|---|---|---|---|---|---|---|
| Met | Tyr | Gly | Gly | Phe | Leu | Arg | Lys | Tyr | Pro | Lys | stop | stop |

Hind III

←—— F1 ——→ ←——— F2 ———→ ←——— F3 ———→ ←——— F4 ——→

```
AGCTTCATGTATGGCGGTTTCCTTCCTAAGTATCCGAAGTAATAG
AGTACATACCGCCAAAGGAAGCATTCATAGGCTTCATTATCCTAG
```

←——— F8 ———→ ←——— F7 ———→ ←——— F6 ———→ ←— F5

BamHI

F-1  5′AGCTTCATGT              F-5  5′GATCCTATT 3′

F-2  ATGGCGGTTTCC             F-6  ACTTCGGATACT

F-3  TTCGTAAGTATC             F-7  TACCAAGGAAAC

F-4  CGAAGTAATAG              F-8  CGCCATACATGA

Hind III

| Pro | Glu | Ser | Phe | Met | Tyr |
|---|---|---|---|---|---|
| ----- CCCGAA | | AGC | TTC | ATG | T---- |
| ----- GGGCTT | TCG | AAG | TAC | A---- | |

←——— Trp1 gene ———→ ←——— αNEgene ———→

1/9

0077689

FIG. 2

# FIG. 3

# FIG. 4

# FIG.5

FIG.6

FIG. 7

FIG. 8

# FIG.9